# FASCICULE DE BREVET EUROPEEN

(11) **EP 3 084 515 B1**
(45) Date de publication et mention de la délivrance du brevet: **29.03.2023**
(21) Numéro de dépôt: 14827470.7
(22) Date de dépôt: 03.12.2014
(51) Int. Cl.: G02C 13/00

(54) **PROCEDE DE CONTROLE DE LA CONFORMITE DE MONTAGE D'UNE MONTURE ET DE VERRES CORRECTEURS**
VERFAHREN ZUR ÜBERPRÜFUNG DER EINWANDFREIEN MONTAGE EINES RAHMENS UND KORREKTURLINSEN
METHOD FOR CHECKING THE CORRECT ASSEMBLY OF A FRAME AND CORRECTIVE LENSES

(30) Priorité: 16.12.2013 FR 1362717
(43) Date de publication de la demande: 26.10.2016
(73) Titulaire: Essilor International, 94220 Charenton-le-Pont (FR)
(72) Inventeur: PETIGNAUD, Cécile, F-94800 Villejuif (FR); LALOUX, Thierry, F-75020 Paris (FR); LAKHCHAF, Nacer, F-60100 Creil (FR); ROUSSEAU, Benjamin, F-91700 Villiers-sur-orge (FR); BERTHEZENE, Marie-Anne, F-94700 Maison-alfort (FR)
(74) Mandataire: Ipsilon
(86) Numéro de dépôt international: PCT/FR2014/053146
(87) Numéro de publication internationale: WO 2015/092194

(56) Documents cités:
- WO-A1-99/01791
- FR-A1- 2 896 682
- FR-A1- 2 980 592
- US-A1- 2011 007 269

## Description

L'invention se rapporte à un procédé de contrôle de la conformité de montage d'une monture et de verres correcteurs. Généralement, un tel procédé intervient à la livraison d'une monture munie de verres correcteurs, après que des paramètres morpho géométriques du porteur aient été déterminés lors d'une étape préalable de prises de mesure réalisée, soit par un opticien soit par le porteur lui-même.

Des documents de mesures de la position de la monture sur le visage de porteur incluent US 2011 /007269 A1, FR 2 896 682 A1, FR 2 980 592 A1, WO 99/01791 A1.

Actuellement, cette étape préalable de prises de mesure est effectuée par un opticien au moyen de différents équipements, pouvant par exemple être constitué d'un pupillomètre à reflets cornéens pour déterminer la distance interpupillaire, d'un réglet, d'un mesureur de hauteur de pupilles. Une fois que ces principaux paramètres ont été évalués, une monture avec les verres correcteurs adaptés est commandée, puis livrée. L'opticien utilise couramment une carte de centrage et un réglet pour contrôler le centrage des verres dans l'équipement. L'horizontalité des micro-cercles est évaluée visuellement. Ces contrôles peuvent être effectués, soit de façon autonome sur l'équipement seul, soit sur l'équipement lorsque celui-ci est en position fonctionnelle sur un individu.

Un inconvénient important de ce procédé de contrôle est que certaines mesures sont subjectives car elles dépendent de l'opticien, et qu'elles subissent des effets de parallaxe, qui augmentent l'incertitude sur lesdites mesures. Lorsqu'un moyen de mesures est utilisé, il a son propre référentiel, qui peut être différent d'un autre moyen de mesure utilisé, accroissant un peu plus les sources d'erreurs sur les mesures. Enfin, les mesures effectuées lors de l'étape préalable et qui permettent d'accéder aux caractéristiques du visage du porteur nécessaires à l'élaboration de l'équipement complet, comprenant monture et verres, dépendent également de la posture de la personne pendant lesdites mesures. Autrement dit, les procédés de contrôle existants présentent un certain nombre d'approximations, susceptibles de multiplier les sources d'erreur et donc de biaiser le diagnostic sur la conformité de montage de la monture et des verres correcteurs.

Les procédés de contrôle de la conformité de montage d'un équipement, composé d'une monture et des verres correcteurs selon l'invention, permettent de proposer un diagnostic clair et réaliste, en s'affranchissant des inconvénients relevés dans l'état de la technique.

L'invention a pour objet un procédé de contrôle de la conformité de montage d'un équipement composé d'une monture et de deux verres ophtalmiques correcteurs conforme à la revendication 1.

Le principe d'un tel procédé de contrôle repose sur des mesures objectives, effectuées à partir d'au moins une image du visage d'un porteur avec l'équipement composé de la monture et des deux verres correcteurs, ledit équipement étant doté de repères de centrage et d'horizontalité pour faciliter une évaluation sûre et rapide de la conformité de montage. Préférentiellement, une fois que la conformité du montage a été vérifiée, ces repères peuvent s'enlever facilement dudit équipement. Ces repères peuvent par exemple être représentés par des traces sur les verres, matérialisant l'emplacement des croix de montage et/ou un axe horizontal, lesdites traces pouvant être réalisées au moyen d'un stylo feutre. L'étape amont peut être réalisée, soit au moyen de techniques spécifiques impliquant différents appareillages adaptés, soit à partir de l'acquisition d'au moins une image du visage du porteur avec une monture achetée par le porteur ou une monture de même référence sans verres correcteurs. L'étape de comparaison permet d'évaluer l'écart qu'il peut y avoir entre chaque paramètre déterminé lors de l'étape amont et chaque paramètre déduit de l'étape aval. Les termes « amont » et « aval » sont à interpréter par rapport au temps, l'étape amont étant ainsi antérieure à l'étape aval. Selon une variante de réalisation d'un procédé de contrôle selon l'invention, l'étape amont de mesure et l'étape aval sont confondues. En effet, sur l'image de l'étape aval, on peut visualiser à la fois les repères de centrage et d'horizontalité de l'étape aval, et les paramètres qu'on détermine pendant l'étape amont. La comparaison est donc faite sur l'image de l'étape aval. Pour être complet un procédé de contrôle selon l'invention doit comporter une étape de conclusion permettant de prendre clairement position par rapport à la conformité de montage de la monture et des verres correcteurs. Cette étape de décision peut s'effectuer, soit de façon rigoureuse et objective par rapport à des écarts de référence prédéterminés sur chaque paramètre, soit au moyen d'une simple visualisation de l'image acquise lors de l'étape aval.

Grâce au fait que l'étape amont est réalisée au moyen de l'acquisition d'au moins une image du visage du porteur avec une monture sans les verres correcteurs, l'étape amont et l'étape aval sont réalisées selon le même principe et permettent de faciliter l'étape de comparaison entre chaque paramètre, en limitant les sources d'erreur dues à des conditions opératoires totalement différentes.

Le fait que l'étape amont et l'étape aval sont réalisées avec le même mode opératoire et avec des moyens de mesure identiques simplifie encore plus l'étape de comparaison entre chaque paramètre, en éliminant les sources d'erreur liées à des conditions de déroulement différentes entre l'étape amont et l'étape aval, et à l'utilisation de moyens de mesure différents pouvant par exemple posséder un étalonnage distinct.

De façon avantageuse, lorsque l'étape amont et l'étape aval sont réalisées dans des référentiels différents, ledit procédé comprend une étape de réajustement des paramètres pour pouvoir effectuer l'étape de comparaison. En effet, en utilisant des référentiels différents liés aux caractéristiques intrinsèques des appareils et matériels retenus pour effectuer l'étape amont et l'étape aval, il est impératif de procéder à une correction de certaines valeurs des paramètres déterminés, afin de ramener toutes les valeurs des paramètres à un même référentiel, et ainsi fiabiliser l'étape de comparaison.

Avantageusement, un procédé selon l'invention, comprend une étape de contrôle de la posture du porteur entre l'étape amont et l'étape aval, pour s'assurer que les positions relatives entre l'équipement et le porteur soient sensiblement identiques lors desdites deux étapes. En effet, la position relative de l'équipement, qui est composé dans un cas par la monture et les verres correcteurs et dans l'autre cas par la monture sans verres correcteurs, ne doit pas être trop différente entre l'étape amont et l'étape aval, sous peine d'introduire une source d'erreurs supplémentaire et significative pouvant biaiser le diagnostic.

De façon préférentielle, l'étape de comparaison porte sur au moins un paramètre géométrico-morphologique à choisir parmi la hauteur, l'angle pantoscopique, l'horizontalité de la monture, l'horizontalité des verres par rapport à ladite monture, l'écart pupillaire, le galbe de ladite monture et la position des repères de centrage sur chacun des verres.

Grâce à l'étape de validation de la conformité de l'équipement composé de la monture munie des verres correcteurs intégrant des seuils de tolérances sur les mesures, même s'il existe des écarts de valeurs entre les paramètres déterminés lors de l'étape amont et ceux mesurés lors de l'étape aval, cela ne signifie pas pour autant que le montage de la monture et des verres correcteurs n'est pas conforme. Il est donc important de pouvoir définir des plages de valeurs, dans lesquelles l'écart entre les paramètres pourra être considéré comme non significatif et permettra donc de valider la conformité de montage de l'équipement.

Selon une caractéristique ne faisant pas partie de l'invention, le procédé de contrôle peut comprendre une étape de présentation de la validation de la conformité, au moyen d'au moins une image du visage du porteur avec la monture de verres correcteurs et d'au moins une information visible sur ladite image. Un procédé de contrôle selon l'invention doit être complet en proposant au porteur une information conviviale et claire sur la conformité de montage de la monture et des verres correcteurs. Un tel procédé n'est pas contraignant, dans la mesure où il n'oblige pas le porteur à effectuer une quelconque action pour accéder au diagnostic sur la conformité dudit montage. L'information peut par exemple apparaitre sous la forme d'une couleur, vert si c'est conforme ou rouge sinon, ou sous la forme de tout autre symbole distinctif permettant de lire la validation sans ambiguïté.

Selon une caractéristique ne faisant pas partie de l'invention, le procédé de contrôle peut comprendre une étape de renseignement d'une base de données dans laquelle apparait pour un porteur et des moyens de mesure identifiés, les paramètres déterminés lors de l'étape amont, les conditions opératoires dans lesquelles ont été obtenus lesdits paramètres et le type de moyens de mesure utilisé, ladite base étant rendue accessible pour chacun desdits porteurs au moyen d'un identifiant. En effet, certaines caractéristiques géométrico-morphologiques du visage d'un porteur telles que l'écart pupillaire évoluant peu avec le temps, il est souhaitable de ne pas avoir à répéter l'étape amont à chaque fois que le porteur désire changer de monture et/ou de verres correcteurs, ce type d'étape constituant toujours une contrainte.

Selon une caractéristique ne faisant pas partie de l'invention, le procédé de contrôle peut comprendre une étape d'ajustement de la mesure. Cette étape permet d'informer la personne qui réalise l'étape aval, que ce soit le porteur ou l'opticien, que le résultat de la mesure ne sera pas exploitable pour valider la conformité de montage de la monture et des verres correcteurs. Cette étape peut constituer une invitation à modifier en temps réel les conditions opératoires. Une fois cette étape d'ajustement de la mesure effectuée, la comparaison entre les mesures de l'étape amont et celles de l'étape aval peut être effectuée. Si ces mesures révèlent un écart supérieur à une valeur seuil, alors une alerte est signalée.

Selon un exemple ne faisant pas partie de l'invention, les étapes amont et aval peuvent être réalisées par le porteur avec des moyens dotés d'un écran et reliés à un réseau de télécommunication. Pour cette configuration de procédé de contrôle, le porteur effectue lui-même et sans l'assistance d'un opticien, les différentes étapes dudit procédé. En effet, il existe actuellement toute une série de moyens électroniques portables, parmi lesquels des ordinateurs, des téléphones ou des tablettes, qui sont dotés d'un appareil photo et/ou d'une webcam permettant de réaliser les différentes étapes d'un procédé de contrôle selon l'invention. Le réseau de télécommunication est avantageusement constitué par le réseau internet.

Selon une caractéristique ne faisant pas partie de l'invention, l'étape aval, l'étape de comparaison et l'étape de validation peuvent être réalisées sur un site dédié de l'internet. Ce site peut par exemple appartenir à un fabricant de montures ou à un fabricant de verres, ou à un organisme dont l'activité est directement liée à la correction de la vue d'un individu. Ledit site peut également proposer une méthodologie conviviale réalisée au moyen de différentes actions à entreprendre par le porteur, pour aboutir à une validation de la conformité de montage de la monture et des verres correcteurs. Un type d'action typique que pourrait réaliser un porteur consisterait à positionner son visage devant l'écran d'un équipement électronique doté d'une webcam, dans un encadrement prédéfini dudit écran et à acquérir différentes images avec et sans la monture et à attendre le verdict.

Selon une caractéristique ne faisant pas partie de l'invention, l'étape aval, l'étape de comparaison et l'étape de validation peuvent être effectuées à partir d'un affichage sur l'écran pendant la prise d'images et d'une instruction lui indiquant comment modifier sa posture dans le but de minimiser les erreurs lors de l'étape de comparaison.

Avantageusement, l'étape de décision sur la conformité de montage comprend une étape de contrôle de la position de repères distinctifs sur les verres correcteurs, ladite étape de contrôle s'effectuant avec un individu portant l'équipement monté.

De façon préférentielle, chaque repère distinctif est une croix apposée sur chaque verre de l'équipement, lesdites croix étant censées matérialiser la position des yeux de l'individu. Ces croix peuvent, par exemple, être apposées temporairement sur les verres avant d'être retirées une fois que l'étape de contrôle a été réalisée. L'étape de contrôle s'effectue avec une certaine tolérance, une divergence minime de positions entre les croix et les yeux de l'individu pouvant être acceptée pour obtenir un diagnostic positif sur la conformité de montage de l'équipement. Cette divergence minime doit toutefois demeurer inférieure à une valeur seuil prédéterminée.

Les procédés de contrôle de la conformité de montage d'une monture et des verres correcteurs selon l'invention présentent l'avantage d'être faciles et rapides à mettre en oeuvre sans imposer au porteur des postures contraignantes. Ils ont de plus l'avantage de proposer une validation de la conformité de montage, qui est sûre et fiable, en raison du caractère objectif des mesures ne dépendant ni d'un opticien ni dudit porteur. Ils présentent enfin l'avantage d'être conviviaux, en proposant une étape d'affichage sur un écran du diagnostic de conformité qui se lit et s'interprète sans ambiguïté.

On donne ci-après, une description détaillée de différents modes de réalisation préférés d'un procédé de contrôle de la conformité de montage d'une monture et de deux verres correcteurs selon l'invention.

Généralement, un individu cherchant à s'équiper d'une monture et de verres correcteurs, se rend chez un opticien avec la prescription d'un ophtalmologiste précisant les caractéristiques optiques des verres correcteurs à monter. L'opticien se livre alors, au moyen de mesures et/ou de calculs, à la détermination d'un certain nombre de paramètres morpho-géométriques du visage de cet individu, comme par exemple les écarts pupillaires, pour pouvoir dimensionner avec le plus de précision possible ladite monture et lesdits verres correcteurs. Une fois que la monture et lesdits verres correcteurs ont été livrés montés, il est indispensable de vérifier la qualité de ce montage pour s'assurer que l'équipement ainsi livré, est conforme aux paramètres morpho-géométriques préalablement déterminés et qu'il ne risque donc pas d'engendrer des désagréments au porteur liés à un montage approximatif voire défectueux.

Deux configurations sont abordées : l'une pour laquelle le procédé se déroule dans un magasin d'optique en présence d'un opticien, et l'autre pour laquelle le procédé se déroule à distance en présence uniquement du porteur.

Abordons d'abord la configuration pour laquelle le procédé selon l'invention se déroule chez un opticien.

Supposons dans un premier temps, que l'opticien possède un appareil de prise d'images et un écran de visualisation de chacune desdites images.

L'étape amont du procédé peut par exemple servir à déterminer la ou les hauteurs des centres de rotation des yeux CRO et les demi-écarts pupillaires. Ainsi, pour de la mesure de ces deux paramètres :
- la personne porte la monture choisie, que l'opticien a pris soin d'ajuster, à ce stade, sans verres correcteurs.
- un système permettant de fournir un étalon de taille et de qualification de l'inclinaison est présent sur une image correspondant à une photo prise de face, ledit système pouvant par exemple être représenté par un clip de type « Visioffice »,
- l'opticien demande à la personne d'adopter une position de référence, pouvant par exemple être une position primaire (position pour laquelle la personne se tient debout confortablement et regarde droit devant elle) ou une posture spécifique connue comme celle permettant la conduite ou la lecture. Cette position ne concerne que la position de la tête.
- la détermination des hauteurs et des demi-écarts pupillaires peut ensuite s'effectuer de deux façons différentes: soit par une mesure directe sur la photo prise de face, soit par une mesure telle que celle qui est préconisée dans le brevet FR2914173.
- Dans les deux cas, on obtient les valeurs qui serviront à centrer les verres dans la monture, à la fois en horizontal à travers les écarts des centres de rotation des yeux CRO ou les écarts pupillaires, et en vertical à travers la hauteur du centre des pupilles.

L'étape aval de mesure va s'effectuer sur le porteur avec la monture et les verres correcteurs livrés. Cette étape se déroule comme suit :
- l'opticien remet à son client l'équipement complet composé de la monture ajustée et des deux verres correcteurs,
- un repère du verre est accessible sur une image correspondant à une photo prise de face du visage du porteur avec l'équipement livré, ledit repère pouvant par exemple être constitué par les points caractéristiques du montage marqués au feutre,
- le même système que celui utilisé lors de l'étape aval, permettant de fournir un étalon de taille et de qualification de l'inclinaison, est présent sur l'image correspondant à une photo prise de face. Selon une variante de réalisation, un clip peut également être utilisé à condition d'en connaitre les dimensions.
- l'opticien demande à son client de se positionner dans la position de référence, comme lors de l'étape amont précédente ;
- puis lui demande de regarder droit devant;
- l'opticien déclenche une photo ou enregistre un film.

L'étape de comparaison des paramètres entre l'étape amont et l'étape aval, est réalisée au moyen d'un traitement d'image permettant de situer les points de feutre sur le verre, et donc la position de ces points dans la monture, et également de situer, soit les reflets cornéens des deux yeux, soit les centres de pupille des deux yeux. L'objectif de ce traitement d'image est de vérifier que la croix de montage pointée au feutre est bien superposée aux reflets cornéens, ou au(x) centre(s) de(s) pupille(s) des deux yeux.

Supposons dans un deuxième temps, que l'opticien possède un appareil de type « Visioffice » ou un autre appareil de type caméra ou appareil photo.

L'étape amont, permettant de déterminer la ou les hauteur(s) des centres de rotation des yeux CRO et les demi-écarts pupillaires se déroule comme suit :
- la personne porte la monture choisie, que l'opticien a pris soin d'ajuster, sans ses verres correcteurs
- un clip est disposé sur la monture de la personne pour donner des indications de dimensions, d'inclinaison et de posture du porteur;
- l'opticien demande à la personne de se positionner en position primaire.
- la détermination des hauteurs et des demi-écarts pupillaires peut ensuite s'effectuer par une mesure directe sur la photo prise de face
- On obtient les hauteurs des points pupillaires de chacun des deux yeux au bord inférieur de la monture, ainsi que les demi-écarts pupillaires.

L'étape aval de mesure va s'effectuer sur le porteur avec la monture et les verres correcteurs livrés. Cette étape se déroule comme suit :
- l'opticien remet à son client l'équipement complet composé de la monture ajustée et des deux verres correcteurs ophtalmiques;
- les points caractéristiques du montage du verre dans la monture sont indiqués sur le verre par exemple au feutre;
- le même clip que celui utilisé lors de l'étape amont est disposé sur la monture pour donner des indications de dimensions et d'inclinaison. Le fait d'utiliser le même clip n'est pas une obligation. Dans le cas de l'utilisation d'un clip différent, il suffit de connaitre ses dimensions.
- l'opticien demande à son client de se positionner en position primaire, comme lors de l'étape amont précédente.
- puis lui demande de regarder droit devant;
- une photo ou un film est pris ;

Le cas échéant, l'opticien peut demander au porteur de modifier sa posture pour pouvoir passer à l'étape de comparaison.

L'étape de comparaison des paramètres entre l'étape amont et l'étape aval, est réalisée au moyen d'un traitement d'image permettant de situer les points de feutre sur le verre, et donc la position de ces points dans la monture, et également de situer, soit les reflets cornéens des deux yeux, soit les centres de pupille des deux yeux. L'objectif de ce traitement d'image est de vérifier que la croix de montage pointée au feutre est bien superposée aux reflets cornéens, ou au centre de pupille.

Supposons dans un troisième temps, que l'opticien possède un système complet de mesure de posture par exemple, de type OptiTrack, ou Natural Point, et que la posture du client soit contrôlée.

Lorsque l'opticien demande à la personne de se positionner dans une position de référence, lors de l'étape amont, il convient de faire une mesure de la posture adoptée par le sujet. Cette mesure peut s'effectuer au moyen d'une ou plusieurs photos ou par l'acquisition d'une vidéo. Pour garantir une meilleure précision de l'acquisition de la posture primaire du porteur, des marqueurs sont ajoutés sur la tête dudit porteur et sur le haut du corps, lesdits marqueurs étant indépendants de la monture. L'opticien peut ainsi réaliser une photo ou une vidéo, des éléments du haut du corps de la personne pendant la prise de mesure, qui va permettre de déterminer en position et en orientation les marqueurs dans un référentiel lié au lieu et donc à la posture de la personne. Il est également possible d'utiliser un système de mesure de la posture par rayons infra rouge, comme par exemple de type OptiTrack ou Natural Point) Une solution alternative consiste à ne pas utiliser de marqueurs, et à faire du traitement d'image pour prendre comme référence des éléments morphologiques de référence du visage.

Lors de l'étape aval de mesure des paramètres avec la monture et les verres correcteurs, il est important de garantir que le porteur se trouve dans une posture identique à celle qu'il a adoptée lors de l'étape amont, et en particulier, de garantir une position primaire identique, pour que l'étape de comparaison entre les paramètres déterminés lors de l'étape amont et les paramètres mesurés lors de l'étape aval, soit réalisée de façon crédible, sans introduire de sources d'erreurs supplémentaires. Pour cette étape aval, la détermination de la posture est effectuée avec les mêmes moyens que ceux utilisés lors de l'étape amont, permettant ainsi au porteur de corriger sa posture en temps réel afin de la rendre identique à celle adoptée lors de l'étape amont. L'incitation à corriger sa posture, peut se réaliser au moyen d'une interface du système de mesure, ou par des conseils donnés par l'opticien, qui indique les mouvements à faire pour retrouver la bonne position.

Abordons maintenant la configuration ne faisant pas partie de l'invention, pour laquelle le procédé soit réalisé à distance par le porteur lui-même, par exemple au moyen d'une caméra ou d'une webcam.

L'étape amont du procédé peut par exemple servir à déterminer la hauteur de montage, qui est la distance entre le centre de la pupille et le bord inférieur monture, et les demi-écarts pupillaires. Ainsi, pour de la mesure de ces deux paramètres :
- Le porteur ajuste ou non la monture choisie sur son visage selon les recommandations fournies,
- Le porteur porte la monture choisie munie d'un clip ou d'un système d'étalon de taille, et se place devant la caméra ou la webcam. Le porteur peut ainsi se tenir assis ou debout. Il peut fixer un point précis ou fixer une succession de différents points qui sont déterminés de façon à ce que la position du sujet soit la plus naturelle possible,
- Les écarts pupillaires et hauteurs de montage sont alors déterminés par traitement d'une ou plusieurs prises de vues éventuellement moyennées.

A ce stade du procédé, un opticien présent sur le site ou à distance interprète les prises de vue et ajuste si nécessaire les hauteurs de montage. Par exemple, si un ajustage de la monture est nécessaire et possible, lorsqu'elle est par exemple munie de plaquettes réglables, l'opticien peut préconiser un ajustement de ces plaquettes lors de la phase de montage, et dans ce cas, il peut augmenter ou diminuer la hauteur de montage mesurée.

L'étape aval de mesure va s'effectuer sur le porteur avec la monture et les verres correcteurs livrés. Cette étape se déroule comme suit :
- Le porteur porte la monture munie de ses verres correcteurs, et éventuellement du clip ou du système d'étalon de taille de l'étape aval, la monture ayant été ajustée par le porteur lui-même ou lors du montage des verres par le fabricant.
- Les points caractéristiques des verres représentés par les micro-cercles et les croix de centrage peuvent être pointés sur les verres. Ils ne sont pas pointés sur lesdits verres si l'on considère que leur position dans la monture est connue.
- La ou les prises de vues sont réalisées par le porteur selon le même protocole que lors de l'étape amont.

L'étape de comparaison des paramètres entre l'étape amont et l'étape aval, est réalisée au moyen d'un traitement d'image permettant de contrôler la juxtaposition des croix de montage avec le centre des pupilles des deux yeux.

Supposons maintenant non seulement que le procédé soit réalisé à distance par le porteur lui-même, par exemple au moyen d'une caméra ou d'une webcam, mais que la posture dudit porteur soit contrôlée.

Afin de limiter les sources d'erreur, il est recommandé que le porteur adopte la même posture entre l'étape amont de détermination des paramètres et l'étape aval de mesures desdits paramètres.

Si l'on part du principe que la direction du regard du porteur lors de l'étape amont de détermination et lors de l'étape aval de mesure est bien contrôlée car ledit porteur regarde la même cible, il demeure toutefois important de bien replacer la tête du porteur dans la même position et la même orientation, vis-à-vis du système de mesure et d'affichage.

Il convient donc de faire une mesure de la posture adoptée par le porteur. Cette mesure peut s'effectuer au moyen d'une ou plusieurs photos ou par une acquisition vidéo. Pour garantir une meilleure précision de l'acquisition de la posture primaire du porteur, des marqueurs sont ajoutés sur la tête dudit porteur et sur le haut du corps, lesdits marqueurs étant indépendants de la monture. Ces marqueurs peuvent être imprimés par le porteur ou peuvent être commandés à une entreprise ou un organisme spécialisé. Il devient ainsi possible de faire une acquisition en position et orientation des éléments du haut du corps de la personne pendant la prise de mesure. Une solution alternative consiste à ne pas utiliser de marqueurs, et à faire du traitement d'image pour prendre comme référence des éléments morphologiques de référence du visage.

Pour l'étape aval de mesure, il faut garantir que la caméra ou la webcam, est positionnée exactement au même endroit que lors de l'étape amont de prise de mesure. Pour parvenir à cette position identique, il peut être envisagé une phase de validation/calibration avec un objet de taille connue dans le champ de mesure, comme par exemple une carte bancaire.

Lors de l'étape aval de mesure des paramètres avec la monture et les verres correcteurs, il est important de garantir que le porteur se trouve dans une posture identique à celle qu'il a adoptée lors de l'étape amont, et en particulier, de garantir une position primaire identique, pour que l'étape de comparaison entre les paramètres déterminés lors de l'étape amont et les paramètres mesurés lors de l'étape aval, soit réalisée de façon crédible, sans introduire de sources d'erreurs supplémentaires. Pour cette étape aval, la détermination de la posture est effectuée avec les mêmes moyens que ceux utilisés lors de l'étape amont, permettant ainsi au porteur de corriger sa posture en temps réel afin de la rendre identique à celle adoptée lors de l'étape amont. Un bon moyen d'y parvenir consiste à afficher le contour du visage sur l'écran en transparence avec le retour d'image temps réel et d'afficher celui-ci en vert quand la personne arrive à bien faire correspondre le contour mesuré avec l'image temps réel.

Dans toutes les configurations décrites ci-avant, une étape supplémentaire de décision sur la conformité de montage de l'équipement est effectuée automatiquement éventuellement au moyen d'un résultat binaire de type conforme / non conforme. De plus, pour accentuer le caractère convivial du procédé, cette décision sur la conformité de montage pourra peut être affichée sur un écran de visualisation, montrant par exemple le visage du porteur avec la monture et les verres correcteurs assorti d'une forme colorée, verte si le montage est conforme, ou rouge si ledit montage n'est pas conforme.

Dans toutes les configurations décrites ci-avant, il est nécessaire de garder des informations, soit chiffrées comme par exemple les valeurs de hauteur de montage et de position des CRO, soit sous forme d'images ou de films du visage du porteur en position primaire de regard, depuis l'étape amont jusqu'à l'étape de comparaison entre les paramètres déterminés lors de l'étape amont et les paramètres mesurés lors de l'étape aval. Un système informatique pourra être utilisé pour sauvegarder et permettre d'accéder à ces informations. Cette sauvegarde et cet accès aux informations pourra se faire par différents opticiens ou différentes machines. L'intérêt est de pouvoir bénéficier d'un format de données, qui permettra un échange de ces données de manière standardisée. Autrement dit, le résultat des mesures concernant un individu pourra servir à alimenter une base de données, qui pourra être accessible par un opticien ou par le porteur lui-même moyennant l'utilisation d'un code d'accès sous la forme d'un identifiant.

## Revendications

1. Procédé de contrôle de la conformité de montage d'un équipement composé d'une monture et de deux verres ophtalmiques correcteurs, ledit procédé comprenant les étapes suivantes :
- Une étape amont de détermination d'au moins un paramètre permettant de positionner les verres dans la monture et par rapport au visage,
- Une étape aval d'acquisition d'au moins une image du visage dudit porteur avec ledit équipement sur lequel apparaissent des repères de centrage et d'horizontalité,
- Une étape de comparaison entre ledit au moins un paramètre déterminé lors de ladite étape amont et le paramètre correspondant déduit de ladite au moins une image,
- Une étape de décision effectuée automatiquement sur la conformité de montage de l'équipement,
**caractérisé en ce que** l'étape amont est réalisée au moyen de l'acquisition d'au moins une image du visage du porteur avec une monture sans les verres correcteurs, **en ce que** l'étape amont et l'étape aval sont réalisées avec le même mode opératoire et avec des moyens de mesure identiques et **en ce que** ledit procédé comprend une étape de validation de la conformité de l'équipement composé de la monture munie des verres correcteurs, intégrant des seuils de tolérances sur les mesures.

2. Procédé de contrôle selon la revendication 1, **caractérisé en ce que** l'étape amont et l'étape aval sont réalisées dans des référentiels différents, et **en ce que** ledit procédé comprend une étape de réajustement des paramètres pour pouvoir effectuer l'étape de comparaison.

3. Procédé de contrôle selon la revendication 1 ou 2, **caractérisé en ce qu'**il comprend une étape de contrôle de la posture du porteur entre l'étape amont et l'étape aval, pour s'assurer que les positions relatives entre l'équipement et le porteur soient sensiblement identiques lors desdites deux étapes.

4. Procédé de contrôle selon la revendication 1, 2 ou 3, **caractérisé en ce que** l'étape de comparaison porte sur au moins un paramètre géométrico-morphologique à choisir parmi la hauteur, l'angle pantoscopique, l'horizontalité de la monture, l'horizontalité des verres par rapport à ladite monture, l'écart pupillaire, le galbe de ladite monture et la position des repères de centrage sur chacun des verres,.

5. Procédé de contrôle selon la revendication 1, **caractérisé en ce que** l'étape de décision sur la conformité de montage comprend une étape de contrôle de la position de repères distinctifs sur les verres correcteurs, ladite étape de contrôle s'effectuant avec un individu portant l'équipement monté.

6. Procédé de contrôle selon la revendication 5, **caractérisé en ce que** chaque repère distinctif est une croix apposée sur chaque verre de l'équipement, lesdites croix étant censées matérialiser la position des yeux.

## Patentansprüche

1. Verfahren zur Kontrolle der Montagekonformität einer aus einer Fassung und aus zwei ophthalmischen Korrekturgläsern bestehenden Ausrüstung, wobei das Verfahren die folgenden Schritte enthält:
- einen vorgelagerten Schritt der Bestimmung mindestens eines Parameters, der es ermöglicht, die Gläser in der Fassung und bezüglich des Gesichts zu positionieren,
- einen nachgelagerten Schritt der Erfassung mindestens eines Bilds des Gesichts des Trägers mit der Ausrüstung, auf dem Zentrier- und Horizontalitätsmarkierungen erscheinen,
- einen Schritt des Vergleichs zwischen dem mindestens einen im vorgelagerten Schritt bestimmten Parameter und dem entsprechenden von dem mindestens einen Bild abgeleiteten Parameter,
- einen automatisch ausgeführten Schritt der Entscheidung über die Montagekonformität der Ausrüstung,
**dadurch gekennzeichnet, dass** der vorgelagerte Schritt mittels der Erfassung mindestens eines Bilds des Gesichts des Trägers mit einer Fassung ohne die Korrekturgläser durchgeführt wird, dass der vorgelagerte Schritt und der nachgelagerte Schritt mit der gleichen Betriebsweise und mit gleichen Messeinrichtungen durchgeführt werden, und dass das Verfahren einen Schritt der Validierung der Konformität der aus der mit den Korrekturgläsern versehenen Fassung bestehenden Ausrüstung enthält, die Messtoleranzschwellen einbezieht.

2. Kontrollverfahren nach Anspruch 1, **dadurch gekennzeichnet, dass** der vorgelagerte Schritt und der nachgelagerte Schritt in unterschiedlichen Bezugssystemen durchgeführt werden, und dass das Verfahren einen Schritt der Anpassung der Parameter enthält, um den Vergleichsschritt ausführen zu können.

3. Kontrollverfahren nach Anspruch 1 oder 2, **dadurch gekennzeichnet, dass** es einen Schritt der Kontrolle der Positur des Trägers zwischen dem vorgelagerten Schritt und dem nachgelagerten Schritt enthält, um sich zu vergewissern, dass die relativen Positionen zwischen der Ausrüstung und dem Träger während der Schritte im Wesentlichen gleich sind.

4. Kontrollverfahren nach Anspruch 1, 2 oder 3, **dadurch gekennzeichnet, dass** der Vergleichsschritt sich auf mindestens einen geometrisch-morphologischen Parameter bezieht, der auszuwählen ist unter der Höhe, dem pantoskopischen Winkel, der Horizontalität der Fassung, der Horizontalität der Gläser bezüglich der Fassung, dem Pupillenabstand, der Durchbiegung der Fassung und der Position der Zentriermarkierungen auf jedem der Gläser.

5. Kontrollverfahren nach Anspruch 1, **dadurch gekennzeichnet, dass** der Schritt der Entscheidung über die Montagekonformität einen Schritt der Kontrolle der Position von Unterscheidungsmarkierungen auf den Korrekturgläsern enthält, wobei der Kontrollschritt mit einer die montierte Ausrüstung tragenden Person ausgeführt wird.

6. Kontrollverfahren nach Anspruch 5, **dadurch gekennzeichnet, dass** jede Unterscheidungsmarkierung ein auf jedes Glas der Ausrüstung aufgebrachtes Kreuz ist, wobei die Kreuze die Position der Augen kennzeichnen sollen.

## Claims

1. Method for checking the fitting conformity of a piece of equipment composed of a frame and of two corrective ophthalmic lenses, said method comprising the following steps:
- an upstream step of determining at least one parameter allowing the lenses to be positioned in the frame and relative to the face;
- a downstream step of acquiring at least one image of the face of said wearer with said piece of equipment in which appear centring and horizontality reference marks;
- a step of comparing said at least one parameter determined in said upstream step and the corresponding parameter deduced from said at least one image; and
- an automatically performed step of reaching a decision on the fitting conformity of the piece of equipment,
**characterized in that** the upstream step is carried out by means of the acquisition of at least one image of the face of the wearer with a frame without the corrective lenses, **in that** the upstream step and the downstream step are carried out with the same operating mode and with identical measuring means and **in that** said method comprises a step of validating the conformity of the piece of equipment composed of the frame equipped with the corrective lenses, integrating tolerance thresholds into the measurements.

2. Checking method according to Claim 1, **characterized in that** the upstream step and the downstream step are carried out in different frames of reference, and **in that** said method comprises a step of readjusting parameters in order to allow the comparing step to be performed.

3. Checking method according to Claim 1 or 2, **characterized in that** it comprises a step of checking the posture of the wearer between the upstream step and the downstream step, in order to make certain that the relative positions between the piece of equipment and the wearer are substantially identical in said two steps.

4. Checking method according to Claims 1, 2 or 3, **characterized in that** the comparing step applies to at least one geometrico-morphological parameter chosen from height, pantoscopic angle, the horizontality of the frame, the horizontality of the lenses relative to said frame, pupillary distance, the face-form angle of said frame and the position of the centring reference marks on each of the lenses.

5. Checking method according to Claim 1, **characterized in that** the step of reaching a decision on the fitting conformity comprises a step of inspecting the position of distinctive reference marks on the corrective lenses, said inspecting step being performed with an individual wearing the fitted piece of equipment.

6. Checking method according to Claim 5, **characterized in that** each distinctive reference mark is a cross affixed to each lens of the piece of equipment, said crosses being supposed to indicate the position of the eyes.
